# EUROPEAN PATENT SPECIFICATION

(11) **EP 3 974 000 B1**
(45) Date of publication and mention of the grant of the patent: **06.12.2023**
(21) Application number: 21196076.0
(22) Date of filing: 10.09.2021
(51) Int. Cl.: A61L 2/07

(54) **STEAM STERILIZER**
DAMPFSTERILISATOR
STÉRILISATEUR À VAPEUR

(30) Priority: 28.09.2020 JP 2020162628
(43) Date of publication of application: 30.03.2022
(73) Proprietor: Nakanishi Inc., Kanuma-shi, Tochigi 322-8666 (JP)
(72) Inventor: TAKAHASHI, Hironori, Tochigi, 3228666 (JP)
(74) Representative: Lewis Silkin LLP

(56) References cited:
- EP-B1- 3 279 593
- CN-A- 108 187 084
- CN-U- 206 777 545
- CN-U- 208 809 121
- CN-U- 209 004 763
- CN-U- 210 331 178
- CN-U- 212 347 215
- JP-A- 2016 064 019

## Description

### TECHNICAL FIELD

The present disclosure relates to a sterilizer.

### BACKGROUND ART

In the related art, JP-A-2002-078778 there is a sterilizer that sterilizes a medical instrument including a hand piece with high-pressure steam. The sterilizer includes a chamber that houses a medical instrument therein, a water supply tank that holds water to be supplied into the chamber, and a heating unit that heats the water supplied into the chamber into steam. The sterilizer sterilizes the medical instrument by generating steam inside the chamber and bringing the chamber into a high temperature and high pressure state. The water supply tank of a sterilizer is generally provided inside a housing, and water supply is performed by opening a front door or a lid.

Among such sterilizers, there is a sterilizer in which a water supply path for supplying water into the chamber is formed at a position above the medical instrument to be sterilized. That is, a water supply port is located above the medical instrument accommodated in the chamber. Further, the water supply path may also serve as a dry air pipe for supplying dry air into the chamber. JP2016064019, CN208809121, CN209004763, CN206 777545, CN210331178, CN108187084, EP3279593 all disclose steam sterilizer comprising a housing, a chamber configured to receive medical instruments to sterilise, and a water tank detachable from the housing.

### SUMMARY OF INVENTION

In the sterilizer in which the water supply tank is provided inside the housing, water supply needs to be performed at a position where the sterilizer is installed, which makes it difficult to perform a water supply operation. For example, it is necessary for an operator to bring the sterilizer to a water supply site. Further, since the water supply tank is often integrated with the housing, maintenance of the water supply tank is not always easy in many cases.

The water supply port is disposed upward of the medical instrument housed in the chamber, the medical instrument may be directly splashed with water at the time of water supply. Further, since the water supply path and the dry air path are the same, water remaining in the water supply path may fall on the medical instrument during a drying process. As a result, it may take a long time to dry the medical instrument. Further, there is a concern that sterilization may be incomplete.

The present disclosure is to provide a sterilizer capable of facilitating a water supply operation and a maintenance operation of a water supply tank. Further, the present disclosure is to provide a sterilizer capable of more reliably drying and sterilizing a medical instrument.

According to the present invention, there is provided a sterilizer according to claim 1.

According to the present disclosure, water replenishment and maintenance of the water supply tank is facilitated.

Preferable features are set out in the remaining claims.

### BRIEF DESCRIPTION OF DRAWINGS

FIG. 1 is a perspective view of a sterilization system including a sterilizer and a drainage tank which are connected via a drainage tube.
FIG. 2 is a perspective view of the sterilizer in a state where a lid is opened upward.
FIG. 3 is a perspective view of the sterilizer in a state where the lid is opened upward and a water supply tank is detached.
FIG. 4 is a perspective view of the sterilizer in a state where the water supply tank is detached.
FIG. 5 is a view showing a front surface of a housing of the sterilizer in a state where the water supply tank is detached.
FIGS. 6A and 6B are perspective views of the water supply tank, in which FIG. 6A is a perspective view as viewed from front, and FIG. 6B is a perspective view as viewed from rear.
FIG. 7 is a perspective view showing an inside of the housing and the chamber in a state where the lid is opened upward.
FIGS. 8A and 8B are a top view and a cross-sectional view of the sterilizer, in which FIG. 8A is the top view and FIG. 8B is the cross-sectional view taken along a line I-I of FIG. 8A.
FIGS. 9A and 9B are a top view and a cross-sectional view of the sterilizer, in which FIG. 9A is the top view and FIG. 9B is the cross-sectional view taken along a line II-II of FIG. 9A.
FIGS. 10A and 10B are a top view and a cross-sectional view of the sterilizer, in which FIG. 10A is the top view and FIG. 10B is the cross-sectional view taken along a line III-III of FIG. 10A.
FIGS. 11A and 11B are a top view and a cross-sectional view of the sterilizer, in which FIG. 11A is the top view and FIG. 11B is the cross-sectional view taken along a line IV-IV of FIG. 11A.
FIGS. 12A and 12B are a top view and a cross-sectional view of the sterilizer, in which FIG. 12A is the top view and FIG. 12B is the cross-sectional view taken along a line V-V of FIG. 12A.
FIG. 13 is a conceptual diagram of a chamber in which hand pieces are housed.
FIG. 14 is a block diagram of the sterilization system.

### DESCRIPTION OF EMBODIMENTS

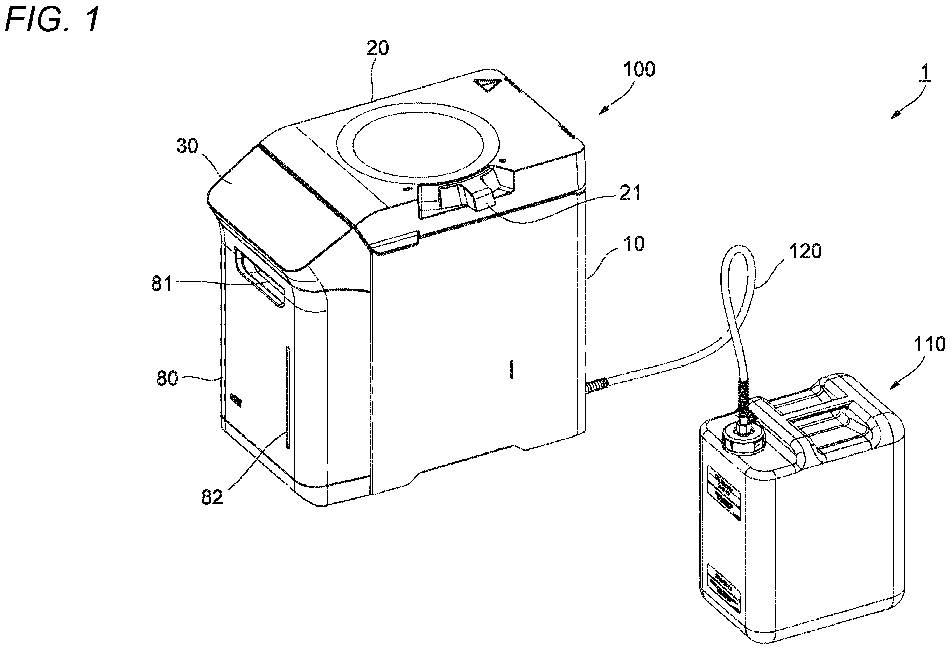

Hereinafter, embodiments of a sterilizer to which the present disclosure is applied will be described in detail with reference to the drawings. FIG. 1 is a perspective view of a sterilization system 1 including a sterilizer 100 and a drainage tank 110 which are connected by a drainage tube 120. The sterilization system 1 is a system for sterilizing a medical instrument including a hand piece used in dental treatment. The medical instrument disposed inside the sterilizer 100 is sterilized by high-temperature and high-pressure water steam, and the water steam and drainage water which is liquefied water steam are sent to the drainage tank 110 via the drainage tube 120.

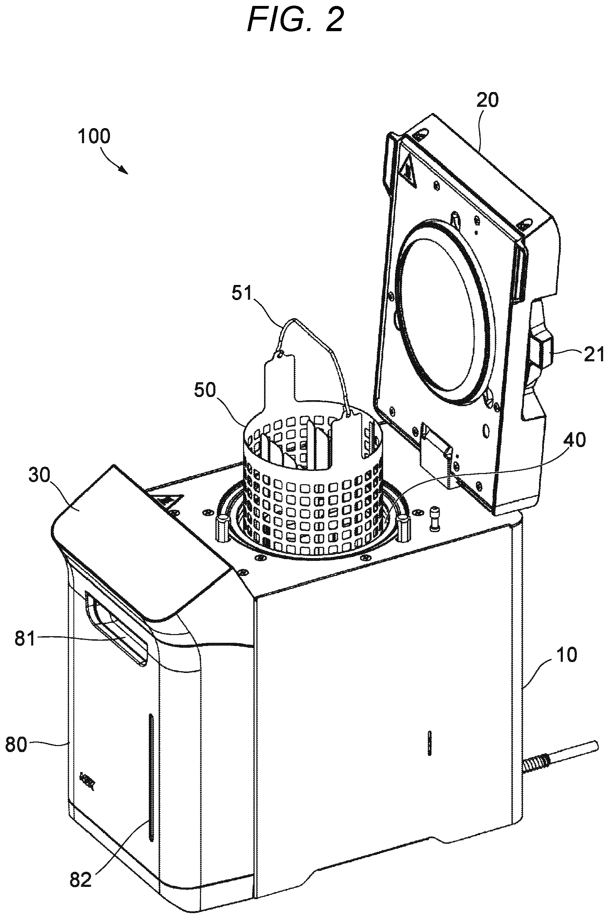

The sterilizer 100 includes a housing 10 that forms an outer shell thereof, a chamber 40 (see FIG. 2) that is disposed inside the housing 10 and houses a medical instrument, and a lid 20 that opens and closes an opening provided above the chamber 40. Further, in the present embodiment, a water supply tank 80 detachably attached to an outer surface, particularly a front surface of the housing 10 is provided. The water supply tank 80 holds water and plays a role of sending water to the housing 10 during a sterilization operation.

As shown in FIG. 2, an operator operates a lid lock lever 21 provided on a side surface of the lid 20 to lift the lid 20 upward, and thus the chamber 40 formed inside the housing 10 is exposed. A housing basket 50 for housing the medical instrument is installed inside the chamber 40, and the operator can pull out the housing basket 50 upward from the chamber 40 while gripping a grip portion 51 of the housing basket 50. FIG. 2 shows a state where the housing basket 50 is pulled out from the chamber 40 halfway.

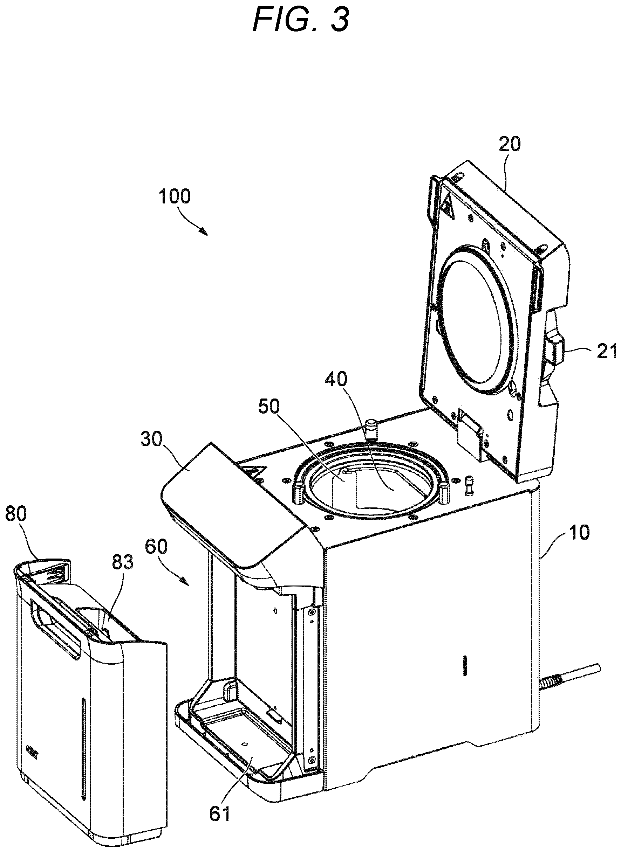

As shown in FIG. 3, a receiving portion 61 protruding forward is provided on a front surface 60 of the housing 10 on a lower side of the housing 10, and an operation panel 30 for operating the sterilizer 100 is provided on the front surface 60 of the housing 10 on an upper side of the housing 10. When the water supply tank 80 is attached to the housing 10, the water supply tank 80 is located below the operation panel 30, and a bottom of the water supply tank 80 is fitted into and supported by the receiving portion 61.

In the sterilizer 100 of the present embodiment, the water supply tank 80 is detachably attached to any of the outer surfaces of the housing 10. By detaching the water supply tank 80, water can be easily supplied and the water supply tank 80 can also be easily maintained. Since the water is supplied into the water supply tank 80 in a state where the operator detaches the water supply tank 80 from the housing 10, the medical instrument is prevented from being splashed with water. An amount of water stored in the water supply tank can also be increased.

In particular, in the present embodiment, since the water supply tank 80 is detachable from the front surface 60 of the housing 10, the water supply tank 80 can be easily attached and detached, and the handleability and the workability is improved.

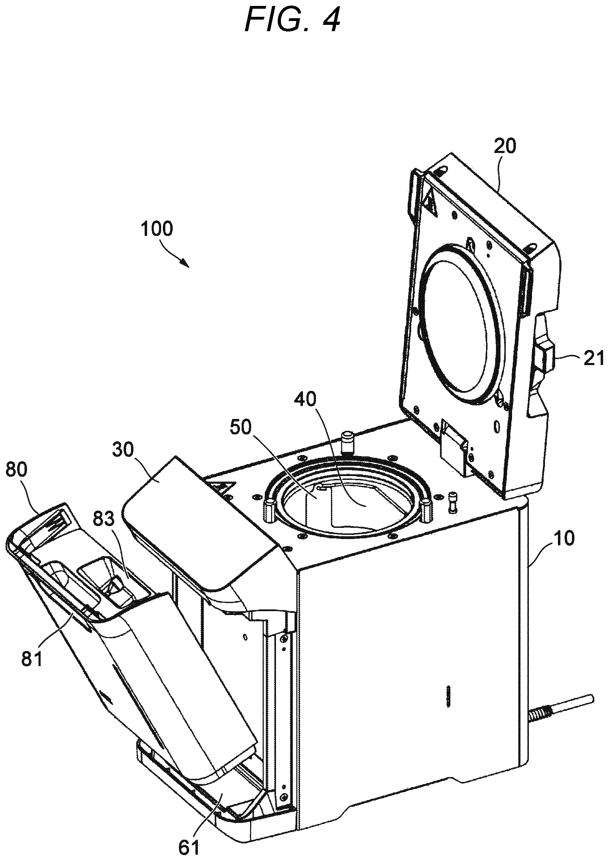

Further, as shown in FIG. 4, when the operator pulls forward a recessed handle portion 81 formed on an upper side of a front surface of the water supply tank 80, the water supply tank 80 is detached from the housing 10 with the receiving portion 61 as a fulcrum. When the water supply tank 80 is to be attached to the housing 10, a reverse operation may be performed. With such a configuration, the water supply tank 80 can be easily and steadily attached and detached. Further, when the water supply tank 80 is detached from the housing 10, the receiving portion 61 supports the water supply tank 80 from below, and the water supply tank 80 rotates around a lower end thereof, so that even if the water supply tank 80 filled with water is heavy, the water supply tank 80 can be easily detached. Further, since there is no need to provide another hinge member, the water supply tank 80 can be attached and detached with a low-cost and simple configuration.

The operation panel 30 is located on a front side of the lid 20, and the water supply tank 80 is located below the operation panel 30. With such a configuration, the sterilizer 100 is made compact, and the design thereof is improved. Further, since the water supply tank 80 is located below the operation panel 30, a state of the water supply tank 80 can be confirmed without largely moving a line of sight from the operation panel 30 when the operator operates the operation panel 30. The state of the water supply tank 80 here is, for example, an attached state of the water supply tank 80 to the housing 10, an amount of water inside the water supply tank 80, and the like.

As shown in FIGS. 3, 4, and FIGS. 6A and 6B, the water supply tank 80 includes a water storage portion 85 having a space capable of storing water, and a water intake port 83 that is opened upward is formed in an upper surface of the water storage portion 85. After detaching the water supply tank 80 from the housing 10, the operator can supply water into the water storage portion 85 from above via the water intake port 83, so that the water can be easily supplied into the water supply tank 80.

A window portion 82 for checking a water level is provided on the front surface of the water supply tank 80. The window portion 82 is made of a transparent resin or the like, and allows an inside of the water storage portion 85 to be visually recognized from the outside. The window portion 82 is located below the water intake port 83, and the operator may check the water level through the window portion 82 in the process of supplying water into the water supply tank 80 that is detached from the housing 10. After the water supply tank 80 is attached to the housing 10, a remaining amount of water may also be checked through the window portion 82.

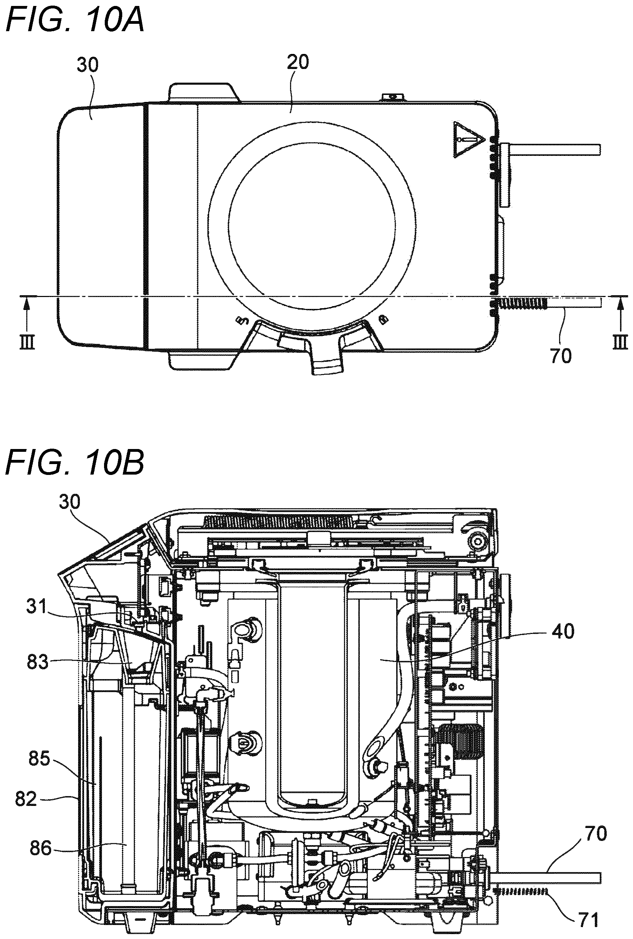

As shown in FIG. 10B, an illumination unit 31 for checking the water level of the water supply tank 80 is provided below the operation panel 30 of the housing 10. The illumination unit 31 is configured by, for example, a light emitting diode (LED), and is located above the water intake port 83 in a state where the water supply tank 80 is attached to the housing 10. Since the illumination unit 31 emits light to passes through the water intake port 83 so as to illuminate a region of the water storage portion 85 where the window portion 82 is provided, confirmation of the water level exposed in the window portion 82 is facilitated.

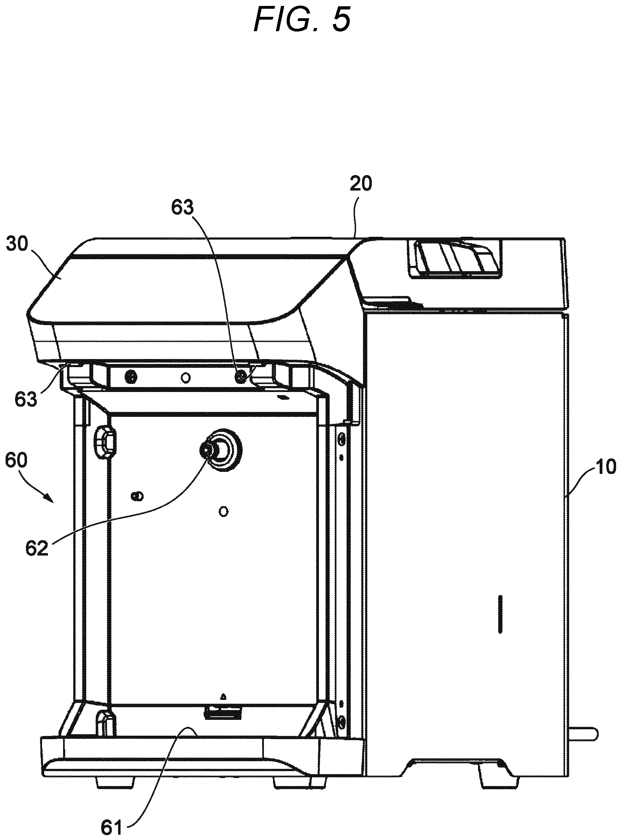

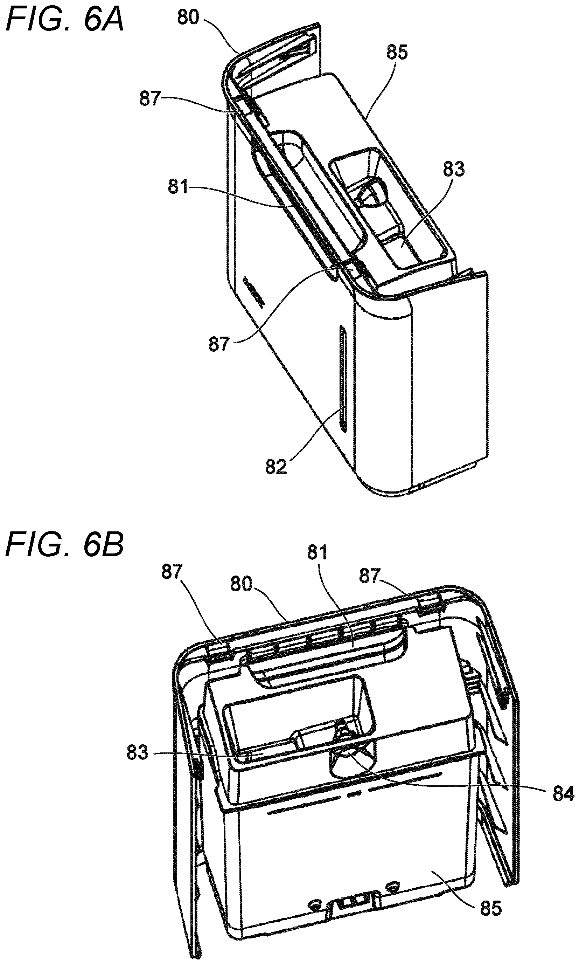

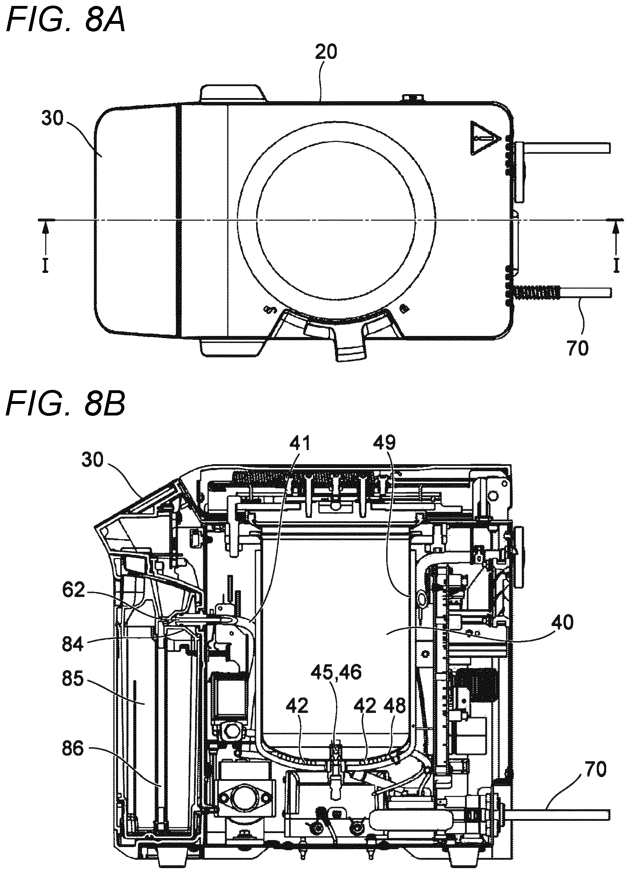

As shown in FIG. 5, a connection end 62 of a water supply pipe 41 (see FIG. 8B) for sending the water in the water supply tank 80 to the chamber 40 is provided on the front surface 60 of the housing 10. Meanwhile, as shown in FIG. 6B, a connection end 84 of a pumping-up pipe 86 (see FIG. 8B) for pumping up the water inside the water supply tank 80 is provided on a rear surface of the water supply tank 80. When the water supply tank 80 is attached to the housing 10, the connection end 84 of the pumping-up pipe 86 and the connection end 62 of the water supply pipe 41 are connected, and water is supplied from the water supply tank 80 to the housing 10. Thus, the water can be easily and reliably supplied from the water supply tank 80 to the housing 10. As described above, the water supply tank 80 is detached from the housing 10 with the lower end thereof as an axis (see FIG. 4), and the connection end 62 of the water supply pipe 41 and the connection end 84 of the pumping-up pipe 86 have a positional relationship corresponding to such an operation. The operator can attach and detach the water supply tank 80 without paying particular attention to alignment.

Spring type locking claws 63 are provided on the front surface of the housing 10. During the attachment operation of the water supply tank 80 described above, fitting recesses 87 (see FIGS. 6A and 6B) provided in the water supply tank 80 and the spring type locking claws 63 are fitted, and the water supply tank 80 is reliably fixed to the housing 10.

As described above, according to the sterilizer 100 of the present embodiment, the water supply tank 80 is detachably attached to any of the outer surfaces of the housing 10, water replenishment of the water supply tank 80 can be easily performed and the water supply tank 80 can also be easily maintained. Since the water is supplied into the water supply tank 80 in a state where the operator detaches the water supply tank 80 from the housing 10, the medical instrument is prevented from being splashed with water. Further, the amount of water stored in the water supply tank 80 can also be increased. In particular, in the present embodiment, since the water supply tank 80 is detachable from the front surface 60 of the housing 10, attachment and detachment of the water supply tank 80 is facilitated, and the handleability and the workability is improved.

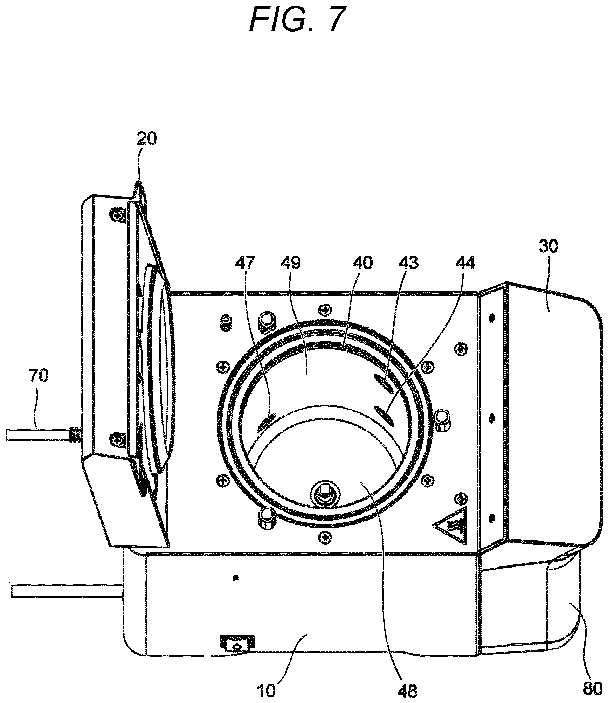

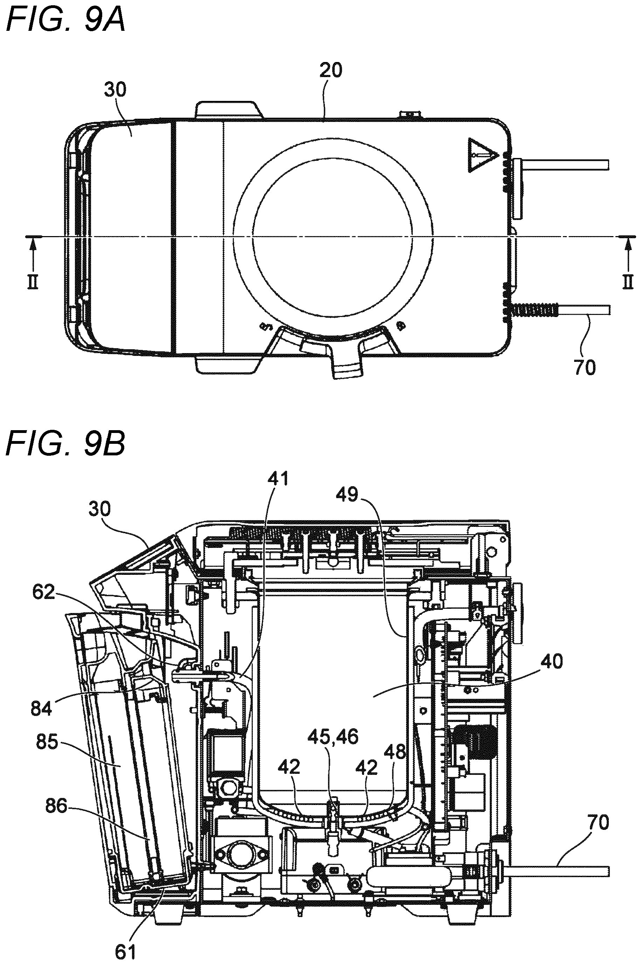

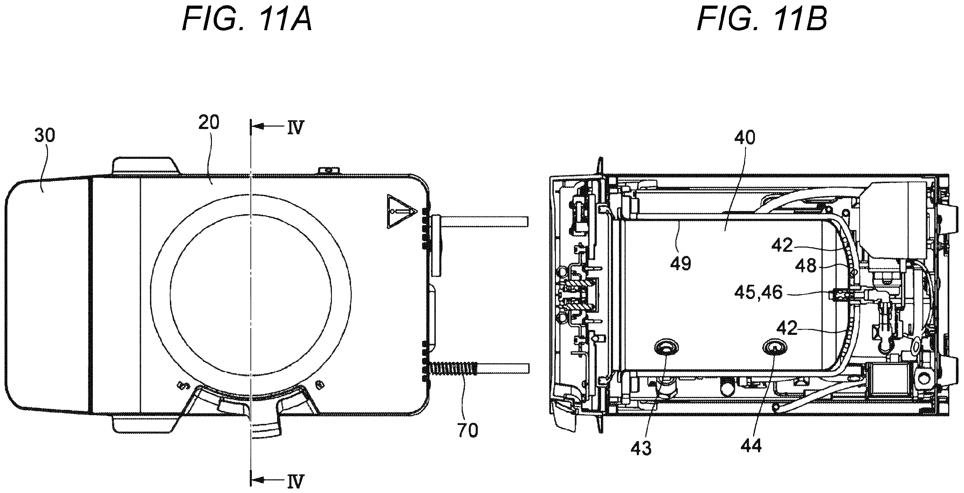

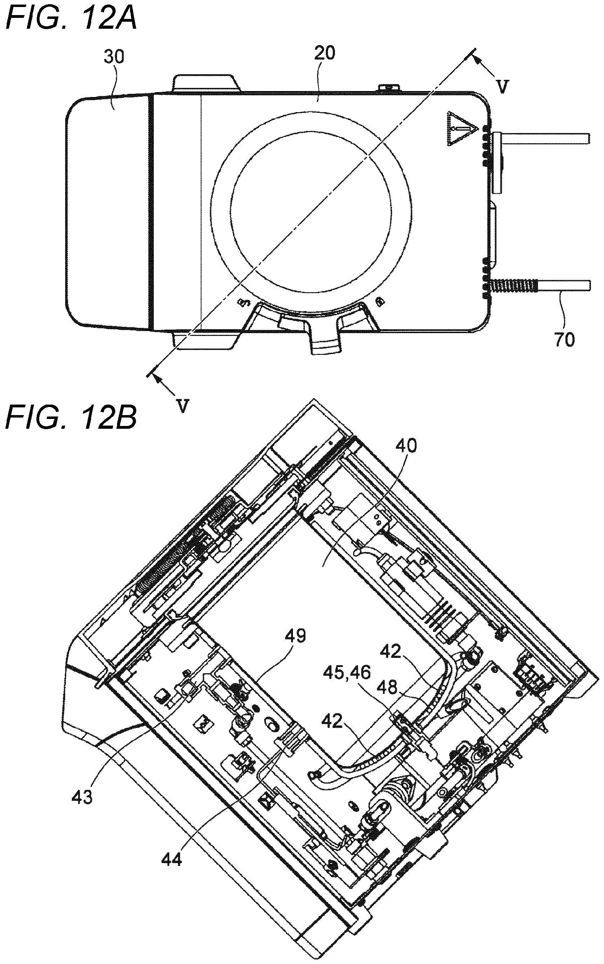

Next, a configuration of the chamber 40 will be mainly described. FIG. 7 shows an inside of the chamber 40 from obliquely above. A power cord 70 for supplying power from the outside extends from the rear surface of the housing 10. FIGS. 8B, 9B, 10B, 11B and 12B show cross sections of the housing 10 and cross sections of the chamber 40 taken along broken lines in FIGS. 8A, 9A, 10A, 11A and 12A. In FIGS. 8B, 9B, 10B, 11B and 12B, the housing basket 50 is not shown.

The chamber 40 has a substantially cylindrical shape, a bottom surface 48 is formed as a dome-shaped curved surface, and a side surface 49 extends upward from the bottom surface 48 into a cylindrical shape. The bottom surface 48 is provided with a water supply port 45 for supplying water to the inside of the chamber 40 and a water drainage port 46 for draining water from the inside of the chamber 40 to the outside. The side surface 49 is provided with an air blowout port 43 for supplying air to the inside of the chamber 40 and an air discharge port 47 for discharging air from the inside of the chamber 40. Further, a heating unit (heater) 42 for heating the water inside the chamber 40 is provided on the bottom surface 48 of the chamber 40.

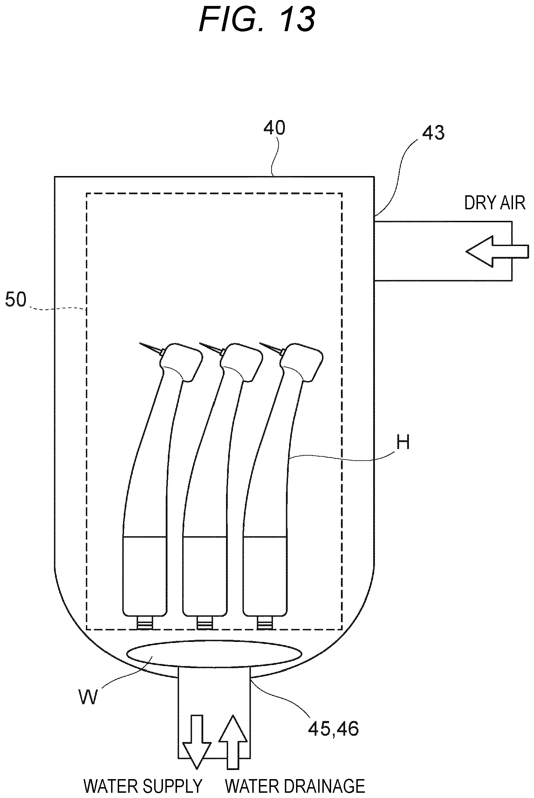

FIG. 13 is a conceptual diagram of the chamber 40 in which hand pieces H which are medical instruments are housed. In FIG. 13, the housing basket 50 is indicated by a broken line. The hand pieces H are housed in a standing state in the housing basket 50. Then, inside the chamber 40, at least the water supply port 45 is formed at a position lower than a housing position of the housed hand pieces H. Further, an amount of water W to be supplied is set such that the water W supplied in a sterilization process is always located below the housing position of the housed hand pieces H.

By forming the water supply port 45 at a position lower than the housing position of the hand pieces H, the water supplied from the water supply port 45 is prevented from directly hitting the hand pieces H. Thus, a dried state of the hand pieces H can be easily maintained, and the sterilization can be quickly performed. In particular, the supplied water is controlled so as to be always located below the housing position of the hand pieces H, so that the dried state of the hand pieces H can be more reliably maintained and the sterilization can be quickly performed.

The air blowout port 43 is formed in a vicinity of an upper portion of the side surface 49, and is formed independently of the water supply port 45 formed in the bottom surface 48. The air blowout port 43 is located above the housing position of the housed hand pieces H. According to such a configuration, the water is prevented from being stored inside the air blowout port 34, so that a situation can be prevented in which the water remaining in the air blowout port 34 is ejected during supply of air from the air blowout port 34 when the hand pieces H are dried. Therefore, water is prevented from hitting the hand pieces H, and the drying performance is improved. Further, the pipe for supplying air and the pipe for supplying water are separated from each other, so that the durability of the pipes can be made different from each other, and the degree of freedom in design can be improved.

In the present embodiment, the water supply port 45 and the water drainage port 46 are formed by the same opening in the bottom surface 48 of the chamber 40. According to such a configuration, the configuration including, for example, sharing a filter to be used is simplified. By forming the water supply port 45 and the water drainage port 46 in the bottom surface 48, water is prevented from directly hitting the hand pieces H.

Further, a temperature detection unit 44 for detecting a temperature inside the chamber 40 is exposed on the side surface 49 of the chamber 40. The temperature detection unit 44 is provided above a position where water is stored inside the chamber 40. The temperature detection unit 44 is prevented from getting wet, so that the temperature can be measured more accurately.

An opening through which the hand pieces H are taken in and out of the chamber 40 faces upward, and the water supply port 45 and the water drainage port 46 are formed in the bottom surface 48 of the chamber 40. Thus, the hand pieces H can be easily taken in and out together with the housing basket 50.

As described above, the medical instruments housed in the chamber 40 are, for example, the hand pieces H used in dentistry and the hand pieces H are housed in a standing state. Thus, drying of the hand pieces H is promoted.

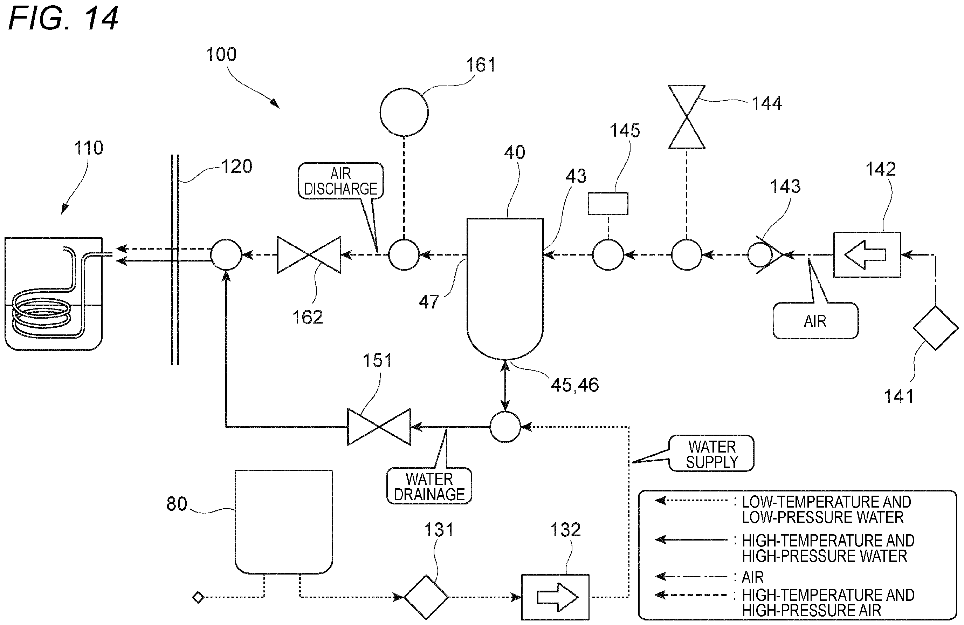

FIG. 14 is a block diagram of the sterilization system 1. From a viewpoint of a system for supplying and discharging a medium (water and air) to and from the chamber 40 that houses the medical instruments, the sterilization system 1 is classified into four systems: (1) a low-temperature and low-pressure water system, (2) a high-temperature and high-pressure water system, (3) an air system, and (4) a high-temperature and high-pressure air system. In FIG. 14, the systems are represented by different lines.

(1) The low-temperature and low-pressure water system is a system for supplying normal water, and the water is supplied along a flow having an order of the water supply tank 80, a water filter 131, and a water pump 132. (2) The high-temperature and high-pressure water system is a system for supplying water pressurized by the water pump 132 and discharging high-temperature and high-pressure water and water steam from the chamber 40. First, the water is supplied along a flow having an order of the water pump 132, the water supply port 45 and the chamber 40. The water is heated by the heating unit 42 in the chamber 40 and vaporized to sterilize the medical instruments housed in the chamber 40.

The high-temperature and high-pressure water remaining after completion of the sterilization process is drained to the drainage tank 110 via the water drainage port 46 passing through a water drainage valve 151 and the drainage tube 120. When water is drained from the chamber 40, the water drainage valve 151 is opened and the water pump 132 is stopped.

(3) The air system supplies normal dry air along a flow having an order of a bacteria filter 141, a drying pump 142 and a check valve 143. (4) The high-temperature and high-pressure air system adjusts a pressure of the air, that is pressurized by the check valve 143, via a safety valve 144 and a calibration connector 145, supplies the air from the air blowout port 43 to the chamber 40, and dries the medical instruments. Further, after the drying is completed, the high-temperature and high-pressure air is sent from the air discharge port 47 of the chamber 40 to the drainage tank 110 passing through a pressure sensor 161, an exhaust valve 162, and the drainage tube 120.

The water is supplied to the chamber 40 by (1) the low-temperature and low-pressure water system and (2) the high-temperature and high-pressure water system, and vaporized so as to sterilize the medical instruments by water steam and drained, and then the air is supplied to the chamber 40 by the air system (3) and the air system (4), so as to dry the medical instruments.

In the present embodiment, since water is supplied and discharged through the water supply port 45 and the water drainage port 46 on the bottom surface of the chamber 40, that is, form below, direct contact of water with the medical instrument is prevented. Further, since the water is not supplied from the water pump 132 to the (3) air system and the (4) high-temperature and high-pressure air system, the water is prevented from being supplied from the air blowout port 43 to the chamber 40 and the medical instrument is smoothly dried.

## Claims

1. A sterilizer (100) comprising:
a housing (10);
a chamber (40) disposed inside the housing and configured to house a medical instrument;
a lid (20) configured to open and close an opening provided above the chamber; and
a water supply tank (80),
wherein the water supply tank (80) is detachably attached to a front surface of the housing (10),
the water supply tank is supported from below by a receiving portion (61) formed on the front surface and a lower side of the housing,
the water supply tank is detachable from the housing with the receiving portion as a fulcrum by pulling forward a handle portion (81) formed on an upper side of a front surface of the water supply tank,
the water supply tank includes a connection end (84) of a pumping-up pipe (86) for pumping up water inside the water supply tank on a rear surface of the water supply tank,
the housing includes a connection end (62) of a water supply pipe (41) for sending water in the water supply tank to the chamber, and
when the water supply tank is attached to the housing, the connection end of the pumping-up pipe and the connection end of the water supply pipe are connected to each other,
and further wherein the connection end of the water supply pipe protrudes along a tangent to a rotation direction of the water supply tank about the fulcrum.

2. The sterilizer (100) according to claim 1 further comprising an operation panel (30) located on a front side of the lid,
wherein the water supply tank (80) is located below the operation panel.

3. The sterilizer (100) according to claim 1 or claim 2, wherein
the water supply tank (80) has a water intake port (83) that is opened upward.

4. The sterilizer (100) according to claim 3, wherein
the water supply tank (80) is provided with a window portion (82) for checking a water level on a front surface of the water supply tank, and
the window portion is located below the water intake port (83).

5. The sterilizer (100) according to claim 3 or 4, wherein
the housing (10) is provided with an illumination unit (31) for checking a water level in the water supply tank (80), and
the illumination unit is located above the water intake port (83) in a state where the water supply tank is attached to the housing.

## Patentansprüche

1. Sterilisator (100), umfassend:
ein Gehäuse (10);
eine Kammer (40), die innerhalb des Gehäuses angeordnet ist und dafür eingerichtet ist, um ein medizinisches Instrument aufzunehmen;
einen Deckel (20), der dafür eingerichtet ist, um eine Öffnung zu öffnen und zu schließen, die über der Kammer bereitgestellt ist; und
einen Wasserzufuhrtank (80),
wobei der Wasserzufuhrtank (80) lösbar an einer Vorderfläche des Gehäuses (10) angebracht ist,
wobei der Wasserzufuhrtank von unten durch einen Empfangsabschnitt (61) getragen wird, der auf der Vorderfläche und einer unteren Seite des Gehäuses gebildet ist,
wobei der Wasserzufuhrtank von dem Gehäuses mit dem Empfangsabschnitt als Drehpunkt gelöst werden kann, indem ein Griffabschnitt (81) nach vorne gezogen wird, der auf einer oberen Seite einer Vorderfläche des Wasserzufuhrtanks gebildet ist,
wobei der Wasserzufuhrtank ein Verbindungsende (84) eines Hochpumprohrs (86) zum Hochpumpen von Wasser innerhalb des Wasserzufuhrtanks auf einer hinteren Fläche des Wasserzufuhrtanks aufweist,
wobei das Gehäuse ein Verbindungsende (62) eines Wasserzufuhrrohrs (41) zum Senden von Wasser in dem Wasserzufuhrtank zu der Kammer aufweist, und
wobei, wenn der Wasserzufuhrtank an dem Gehäuses angebracht ist, das Verbindungsende des Hochpumprohrs und das Verbindungsende des Wasserzufuhrrohrs miteinander verbunden sind,
und wobei ferner das Verbindungsende des Wasserzufuhrrohrs entlang einer Tangente zu einer Drehrichtung des Wasserzufuhrtanks um den Drehpunkt vorsteht.

2. Sterilisator (100) nach Anspruch 1, ferner umfassend ein Bedienerfeld (30), das an einer Vorderseite des Deckels angeordnet ist,
wobei der Wasserzufuhrtank (80) unter dem Bedienerfeld angeordnet ist.

3. Sterilisator (100) nach Anspruch 1 oder Anspruch 2, wobei der Wasserzufuhrtank (80) einen Wassereinlassport (83) aufweist, der nach oben geöffnet ist.

4. Sterilisator (100) nach Anspruch 3, wobei der Wasserzufuhrtank (80) mit einem Fensterabschnitt (82) zum Prüfen eines Wasserstands auf einer Vorderfläche des Wasserzufuhrtanks versehen ist, und
der Fensterabschnitt unter dem Wassereinlassport (83) angeordnet ist.

5. Sterilisator (100) nach Anspruch 3 oder 4, wobei das Gehäuse (10) mit einer Beleuchtungseinheit (31) zum Prüfen eines Wasserstands in dem Wasserzufuhrtank (80) versehen ist, und
die Beleuchtungseinheit über dem Wassereinlassport (83) in einem Zustand angeordnet ist, wo der Wasserzufuhrtank an dem Gehäuse angebracht ist.

## Revendications

1. Stérilisateur (100) comprenant :
un boîtier (10) ;
une chambre (40) disposée à l'intérieur du boîtier et configurée pour abriter un instrument médical ;
un couvercle (20) configuré pour ouvrir et fermer un orifice pratiqué au-dessus de la chambre ; et
un réservoir d'alimentation en eau (80),
le réservoir d'alimentation en eau (80) étant fixé de manière dissociable sur une surface avant du boîtier (10),
le réservoir d'alimentation en eau étant supporté par dessous par une section réceptrice (61) formée sur la surface avant et une face inférieure du boîtier,
le réservoir d'alimentation en eau étant détachable du boîtier avec la section réceptrice sous forme d'un pivot en tirant vers l'avant une partie poignée (81) formée sur une face supérieure d'une surface avant du réservoir d'alimentation en eau,
le réservoir d'alimentation en eau incluant une extrémité de connexion (84) d'un tuyau de pompage (86) pour pomper de l'eau à l'intérieur du réservoir d'alimentation en eau sur une surface arrière du réservoir d'alimentation en eau,
le boîtier incluant une extrémité de connexion (62) d'un tuyau d'alimentation en eau (41) pour envoyer de l'eau du réservoir d'alimentation en eau dans la chambre, et
lorsque le réservoir d'alimentation en eau est fixé au boîtier, l'extrémité de connexion du tuyau de pompage et l'extrémité de connexion du tuyau d'alimentation en eau sont connectées l'une à l'autre,
et l'extrémité de connexion du réservoir d'alimentation en eau saillant en outre le long d'une tangente à un sens de rotation du réservoir d'alimentation en eau autour du pivot.

2. Stérilisateur (100) selon la revendication 1, comprenant en outre un panneau d'actionnement (30) situé sur une face avant du couvercle,
le réservoir d'alimentation en eau (80) se trouvant en dessous du panneau d'actionnement.

3. Stérilisateur (100) selon la revendication 1 ou la revendication 2, dans lequel le réservoir d'alimentation en eau (80) comporte un port d'admission d'eau (83) qui est ouvert vers le haut.

4. Stérilisateur (100) selon la revendication 3, dans lequel le réservoir d'alimentation en eau (80) est pourvu d'une partie fenêtre (82) pour vérifier un niveau d'eau sur une surface avant du réservoir d'alimentation en eau, et
la partie fenêtre se trouve en dessous du port d'admission d'eau (83).

5. Stérilisateur (100) selon la revendication 3 ou 4, dans lequel le boîtier (10) est pourvu d'une unité d'éclairage (31) pour vérifier un niveau d'eau dans le réservoir d'alimentation en eau (80), et
l'unité d'éclairage se trouve au-dessus du port d'admission d'eau (83) dans un état dans lequel le réservoir d'alimentation en eau est fixé au boîtier.
